Europäisches Patentamt

European Patent Office

Office européen des brevets

(19)

(11) Veröffentlichungsnummer: **0 169 977**
**A2**

(12) # EUROPÄISCHE PATENTANMELDUNG

(21) Anmeldenummer: **85104475.0**

(22) Anmeldetag: **12.04.85**

(51) Int. Cl.⁴: **A 61 K 35/78**

(30) Priorität: **31.07.84 DE 3428616**

(43) Veröffentlichungstag der Anmeldung:
**05.02.86 Patentblatt 86/6**

(84) Benannte Vertragsstaaten:
**AT BE CH DE FR GB IT LI LU NL SE**

(71) Anmelder: **KRIWET, Manfred**
**Ansorgestrasse 8**
**D-2000 Hamburg 52(DE)**

(72) Erfinder: **KRIWET, Manfred**
**Ansorgestrasse 8**
**D-2000 Hamburg 52(DE)**

(74) Vertreter: **UEXKÜLL & STOLBERG Patentanwälte**
**Beselerstrasse 4**
**D-2000 Hamburg 52(DE)**

(54) **Verwendung eines diätetischen Lebensmittels bei Darmverstopfung und Darmträgheit sowie Verfahren zur Herstellung des diätetischen Lebensmittels.**

(57) Beschrieben wird ein diätetisches Lebensmittel, das sich zur Anwendung bei Darmverstopfung und Darmträgheit eignet und aus getrockneten Hagebutten, roten Johannisbeeren, weißen Johannisbeeren, Zitronenschalen, Grapefruchtschalen und/oder Orangenschalen besteht. Vorzugsweise liegt das diätetische Lebensmittel in Form von Tabletten, Granulat oder Würfeln vor.

EP 0 169 977 A2

Croydon Printing Company Ltd

**UEXKÜLL & STOLBERG**
PATENTANWÄLTE

BESELERSTRASSE 4
D-2000 HAMBURG 52

EUROPEAN PATENT ATTORNEYS

0169977

DR. J.-D. FRHR. von UEXKÜLL
DR. ULRICH GRAF STOLBERG
DIPL.-ING. JÜRGEN SUCHANTKE
DIPL.-ING. ARNULF HUBER
DR. ALLARD von KAMEKE

– 1 –

Manfred Kriwet
Ansorgestraße 8
2000 Hamburg 52

(21713/KA/wo)

April 1985

## Verwendung eines diätetischen Lebensmittels bei Darmverstopfung und Darmträgheit sowie Verfahren zur Herstellung des diätetischen Lebensmittels

Auf dem Markt befindliche diätetische Lebensmittel zur Verwendung bei Darmverstopfung und Darmträgheit bestehen ausschließlich ober überwiegend aus Weizenkleie in Form von Tabletten, Flocken, Granulat oder ähnlichem und sind gelegentlich durch Zugabe von Rosinen, Nüssen oder Haferflocken sowie manchmal auch Trockenpflaumen geschmacklich verbessert, wobei gleichzeitig eine Verschlechterung des Wirkungsgrades der Weizenkleie in Kauf genommen werden muß. Ferner eignen sich für diätetische Lebensmittel zur Verwendung bei Darmverstofpung und Darmträgheit auch quellstoffreiche aber nährstoffarme Lebensmittel, deren Zahl jedoch gering und deren Verwendung durch die Diätverordnung oder Zusatzstoff-Zulassungsverordnung mengenmäßig begrenzt ist. Bis auf das Gummi arabicum sind diese Quellmittel wie Agar-Agar, Alginate, Guarkernmehl, Pektine und Carboxymethylzellulose nur in Mengen von 0,2 bis 0,3 % in Lebensmitteln zugelassen. Gummi arabicum läßt sich aber verständlicherweise auch nicht unbeschränkt in ein Lebensmittel einarbeiten, da dies zu einem vollständigen Verkleben des Lebensmittels und damit zur Ungenießbarkeit führen würde.

Es wurde nun überraschend gefunden,, daß sich bestimmte getrocknete Früchte bzw. bestimmte getrocknete Teile von Früchten sehr gut als diätetische Lebensmittel zur Verwendung bei Darmverstopfung und Darmträgheit eignen.

Dementsprechend betrifft die Erfindung die Verwendung von getrockneten Hagebutten, roten Johannisbeeren, weißen Johannisbeeren, Zitronenschalen, Grapefruchtschalen, Tangerinenschalen und/oder Orangeschalen als diätetisches Lebensmittel bei Darmverstopfung und Darmträgheit.

Vorzugsweise werden die getrockneten Hagebutten, roten Johannisbeeren, weißen Johannisbeeren, Zitronenschalen, Grapefruchtschalen, Tangerinenschalen und/oder Orangenschalen zu Tabletten, Granulat oder Würfeln verarbeitet und in dieser Form als diätetisches Lebensmittel bei Darmverstopfung und Darmträgheit verwendet.

Die Herstellung der getrockneten Hagebutten, der getrockneten roten Johannisbeeren, der getrockneten weißen Johannisbeeren, der getrockneten Zitronenschalen, der getrockneten Grapefruchtschalen (Pampelmusenschalen), der getrockneten Tangerinenschalen und der getrockneten Orangenschalen, die als Ausgangsmaterial zur Herstellung des diätetischen Lebensmittels dienen, erfolgt üblicherweise durch Luft- oder Ofentrocknung. Vorzugsweise werden reife Früchte eingesetzt (die Orangenschalen stammen meistens aus der Orangensaftherstellung, wo sie als Abfallprodukt anfallen). Der bevorzugte Temperaturbereich für die Trocknung liegt bei 35 bis 50 und insbesondere 40 bis 45$^{\circ}$C. Getrocknet wird

0169977

- 3 -

in der Regel auf einen Restfeuchtigkeitsgehalt von 10 bis 15 Gew.%. Getrocknete Hagebutten, getrocknete Johannisbeeren, getrocknete Zitronenschalen, getrocknete Grapefruchtschalen, getrocknete Tangerinenschalen und getrocknete Orangenschalen sind im Handel erhältlich bzw. können leicht in herkömmlicher Weise erhalten werden. Bezüglich der Hagebutten sei darauf hingewiesen, daß diese für die erfindungsgemäßen Zwecke bevorzugt ohne Samen getrocknet werden.

Das getrocknete Ausgangsmaterial wird in üblichen Mühlen (z.B. Kreuzschlagmühlen) vermahlen. Dabei ist darauf zu achten, daß die Korngröße nicht auf weniger als etwa 1 mm absinkt. Gewöhnlich wird eine Korngröße von 1 bis 2 mm gewählt.

Im nächsten Verfahrensschritt wird das getrocknete und vermahlene Ausgangsmaterial mit einer Quellmittellösung versetzt, so daß eine Mischung mit pastenartiger Konsistenz erhalten wird. Als Quellmittel eignet sich insbesondere Gummi arabicum. Es können aber auch andere Quellmittel, wie Tragant, Agar-Agar, Pektin und Carboxymethylzellulose verwendet werden, wobei allerdings zu beachten ist, daß diese als Zusatzstoffe in der mengenmäßigen Verwendung begrenzt sind (siehe oben).

Die erhaltene pastanartige Mischung wird dann in herkömmlicher Weise mit Hilfe von Granuliermaschinen granuliert. Man kann die Mischung aber auch durch Siebe mit entsprechender Maschenweite pressen. Granulate mit einer Korngröße von etwa 3 mm sind bevorzugt. Das Granulat wird dann getrocknet, was in herkömmlicher Weise auf Horden in Trockenöfen bei Temperaturen von etwa 40 bis 45$^{\circ}$C erfolgen kann. Wenn das diätetische Lebensmittel in Form von Tabletten hergestellt werden soll, wird das getrocknete oder teilgetrocknete Granulat nach herkömmlichen Verahren unter Verwendung üblicher Vorrichtungen zu Tabletten verpreßt.

Soll das diätetische Lebensmittel in Form von Würfeln (bekannt als Fruchtwürfel) hergestellt werden, wird das getrocknete Ausgangsmaterial zusammen mit Rosinen und/oder Trockenfeigen zu einer pastenartigen Mischung vermahlen und in herkömmlicher Weise und unter Verwendung herkömmlicher Vorrichtungen verarbeitet.

Selbstverständlich kann das diätetische Lebensmittel auch in anderen Darreichungsformen wie beispielsweise Flocken hergestellt werden. Bevorzugt sind jedoch Tabletten, Würfel und Granulat. Die Vorteile dieser Darreichungsformen für die praktische Anwendung liegen auf der Hand. Außerdem kann das diätetische Lebensmittel selbstverständlich übliche Zusätze enthalten.

Es hat sich gezeigt, daß die erfindungsgemäße Verwendung von getrockneten Hagebutten, roten Johannisbeeren, weißen Johannisbeeren, Zitronenschalen, Grapefruchtschalen, Tangerinenschalen, Orangenschalen oder Mischungen derselben als diätetisches Lebensmittel bei Darmverstopfung und Darmträgheit gegenüber der herkömmlichen Verwendung von Weizenkleie vorteilhaft ist. Häufig brauchen im Vergleich zu Weizenkleie nur geringere Mengen verzehrt zu werden, um die gleiche Wirkung zu erzielen. In der Regel braucht ein auf getrockneten Hagebutten basierendes diätetisches Lebensmittel nur in einer halb so großen Menge wie Weizenkleie verwendet zu werden. Bei einem diätetischen Lebensmittel auf Basis von getrockneten weißen Johannisbeeren reicht oft sogar schon ein Drittel der erforderlichen Weizenkleiemenge, um die gleiche Wirkung zu erzielen. Hinzu kommt, daß Weizenkleie von vielen Menschen aus geschmacklichen Gründen nur sehr ungern verzehrt oder sogar ganz abgelehnt wird. Die Verwendung von diätetischen Lebensmitteln auf Basis getrockneter Hagebutten, roter Johannisbeeren, weißer Johannisbeeren, Zitronenschalen, Grapefruchtschalen, Tangerinenschalen und/oder Orangenschalen erweitert das Geschmacksspektrum erheblich und erhöht damit die Akzeptanz dieser diätetischen Lebensmittel beim Verbraucher. Darüber hinaus bieten die erfindungsgemäß verwendeten Ausgangsmaterialien weitere spezielle Vorteile, wie beispielsweise den hohen Vitamin C-Gehalt von Hagebutten und Johannisbeeren. Durch den Säure- und Pektingehalt von Johannisbeeren, Hagebutten, Zitronenschalen, Grapefruchtschalen, Tangerinenschalen und Orangenschalen ist darüber hinaus die abführende Wirkung der erfindungsgemäß verwendeten diätetischen Lebensmittel noch weiter gesteigert (vgl. Römpp's Chemielexikon, 7. Auflage, Band 1, Seite 21, Stichwort: Abführmittel).

Die erfindungsgemäß verwendeten diätetischen Lebensmittel können selbstverständlich auch auf Mischungen der verschiedenen geeigneten Ausgangsmaterialien basieren. Durch Variation des prozentualen Anteils der einzelnen Bestandteile kann der Geschmack in jeder beliebigen Weise verändert werden. So können die diätetischen Lebensmittel beispielsweise aus 30 Gew.% getrockneten Johannisbeeren, 30 Gew.% getrockneten Hagebutten und 40 Gew.% getrockneten Orangenschalen, Zitronenschalen, Grapefruchtschalen und/oder Tangerinenschalen oder aus 50 Gew.% getrockneten Hagebutten, 30 Gew.% getrockneten Johannisbeeren und 20 Gew.% Orangenschalen, Zitronenschalen, Grapefruchtschalen und/oder Tangeringenschalen oder 70 Gew.% getrockneten Hagebutten und 30 Gew.% getrockneten Orangeschalen, Zitronenschalen, Grapefruchtschalen und/oder Tangerinenschalen bestehen.

## Beispiel 1

Hagebutten, die ohne Samen getrocknet worden waren, wurden auf eine Korngröße von 1 bis 2 mm vermahlen, mit 2 %iger Gummi arabicum-Lösung vermischt, auf eine Korngröße von 3 mm granuliert, getrocknet und verpackt. Als geeignete Tagesdosis des so hergestellten Granulats haben sich 1 bis 2 Eßlöffel voll, die mit Flüssigkeit verzehrt werden, erwiesen.

## Beispiel 2

Eine Mischung aus 50 Gew.% getrockneten roten Johannisbeeren mit 30 Gew.% getrockneten Hagebutten unter Zusatz von 20 Gew.% getrockneten Orangenschalen wurde auf eine Korngröße von 1 bis 2 mm vermahlen, mit 2 %iger Gummi arabicum-Lösung vermischt und auf eine Korngröße von 3 mm granuliert. Nach dem Trocknen wurde das Granulat zu Tabletten von 2 bis 3 mm verpreßt. Der Verzehr von 4 bis 6 Tabletten hat sich als geeignete Tagesdosis erwiesen.

Anstelle der roten Johannisbeeren wurden auch weiße Johannisbeeren und Mischungen von roten und weißen Johannisbeeren in gleicher Weise eingesetzt und führten zu praktisch gleichen Produkten.

## Beispiel 3

50 Gew.% getrocknete Hagebutten, 30 Gew.% getrocknete Orangenschalen und 20 Gew.% Trockenfeigen wurden vermahlen, bis eine Masse mit pastenartiger Konsistenz erhalten wurde. Diese Masse wurde auf eine Dicke von 1,5 cm ausgewalzt. Dann wurden in üblicher Weise Würfel aus der ausgewalzten Masse ausgestochen. Es erwies sich als zweckmäßig, die Würfel bei 40 bis 45°C nachzutrocknen. In anderen Versuchen wurde die erhaltene Masse auf eine Dicke von 1 cm bzw. von 2 cm ausgewalzt, so daß Würfel mit entsprechend veränderten Abmessungen erhalten wurden.

## Beispiel 4

Eine Mischung aus 60 Gew.% getrockneten Hagebutten und 40 Gew.% getrockneten Grapefruchtschalen wurde auf eine Korngröße von 1 bis 2 mm vermahlen, mit 2 %iger Gummi arabicum-Lösung vermischt, auf eine Korngröße von etwa 2,5 bis 3 mm granuliert und getrocknet. Der Verzehr von 2 bis 3 Eßlöffel voll des Granulats ist eine geeignete Tagesdosis.

## Beispiel 5

Eine Mischung aus 30 Gew.% getrockneten Hagebutten, 30 Gew.% getrockneten roten Johannisbeeren, 30 Gew.% getrockneten Orangenschalen und 10 Gew.% getrockneten Zitronenschalen wurde auf eine Korngröße von 1 bis 2 mm vermahlen, mit einer 2 %igen Carboxymethylzellulose-Lösung vermischt und auf eine Korngröße von 2,5 bis 3 mm granuliert und getrocknet. Die Masse wurde zu Tabletten von 2 bis 3 g verpreßt. Der Verzehr von 4 bis 6 Tabletten ist eine geeignete Tagesdosis.

<u>Ansprüche</u>

1. Verwendung von getrockneten Hagebutten, roten Johannisbeeren, weißen Johannisbeeren, Zitronenschalen, Grapefruchtschalen, Tangerinenschalen und/oder Orangenschalen als diätetisches Lebensmittel bei Darmverstopfung und Darmträgheit.

2. Verwendung nach Anspruch 1, **dadurch gekennzeichnet,** daß die getrockneten Hagebutten, roten Johannisbeeren, weißen Johannisberen, Zitronenschalen, Grapefruchtschalen, Tangerinenschalen und/oder Orangenschalen zu Tabletten, Granulat oder Würfeln verarbeitet sind.

3. Verwendung nach Anspruch 2, **dadurch gekennzeichnet,** daß die Tabletten oder das Granulat zusätzlich ein Quellmittel, insbesondere Gummi arabicum enthalten.

4. Verwendung nach Anspruch 2, **dadurch gekennzeichnet,** daß die Würfel zusätzlich Rosinen und/oder Trockenfeigen enthalten.

5. Verfahren zur Herstellung eines diätetischen Lebensmittels zur Verwendung bei Darmverstopfung oder Darmträgheit in Tabletten- oder Granulatform, **dadurch gekennzeichnet,** daß man getrocknete Hagebutten, rote Johannisbeeren, weiße Johannisbeeren, Zitronenschalen, Grapefruchtschalen, Tangerinenschalen und/oder Orangenschalen vermahlt, mit einer Quellmittellösung zu einer pastenartigen Mischung vermischt und die so erhaltene Mischung granuliert und trocknet oder nach dem Granulieren und Trocknen zu Tabletten verpreßt.

6. Verfahren zur Herstellung eines diätetischen Lebensmittels zur Verwendung bei Darmverstopfung und Darmträgheit in Form von Würfeln, **dadurch gekennzeichnet,** daß man getrocknete Hagebutten, rote Johannisbeeren, weiße Johannisbeeren, Zitronenschalen, Grapefruchtschalen, Tangerinenschalen und/oder Orangenschalen zusammen mit Rosinen und/oder Trockenfeigen zu einer pastenartigen Mischung vermahlt und zu Würfeln verarbeitet.